# EUROPÄISCHE PATENTSCHRIFT

(11) **EP 0 675 095 B1**
(45) Veröffentlichungstag und Bekanntmachung des Hinweises auf die Patenterteilung: **24.03.1999**
(21) Anmeldenummer: 95103583.1
(22) Anmeldetag: 13.03.1995
(51) Int. Cl.: C07C 17/14, C07C 22/04, B01J 19/12

(54) **Verfahren zur Herstellung von 2,2'-Bis(halogenmethyl)-1,1'-binaphthyl**
Process for preparing 2,2'-bis(halogenomethyl)-1,1'-binaphthalene
Procédé pour la préparation de 1,1'Binaphtyl-2,2'-bis halogénométhyle

(30) Priorität: 23.03.1994 DE 4409974; 19.09.1994 DE 4433296
(43) Veröffentlichungstag der Anmeldung: 04.10.1995
(73) Patentinhaber: HOECHST AKTIENGESELLSCHAFT, 65929 Frankfurt am Main (DE)
(72) Erfinder: Kleiner, Hans-Jerg, Dr., D-61476 Kronberg (DE); Regnat, Dieter, Dr., D-65817 Eppstein (DE); Röschert, Horst, Dr., D-55437 Ober-Hilbersheim (DE)

(56) Entgegenhaltungen:
- TETRAHEDRON LETTERS, Bd. 30, Nr. 27, 1989 Seiten 3513-3516, S. L. COLLETTI AND R. L. HALTERMAN 'Binaphthylcyclopentadiene: A C2-Symmetric Annulated Cyclopentadienyl Ligand with Axial Chirality'

## Beschreibung

Die vorliegende Erfindung betrifft ein verbessertes Verfahren zur Herstellung von 2,2'-Bis(halogenmethyl)-1,1'-binaphthyl, insbesondere von 2,2'-Bis(chlormethyl)-1,1'-binaphthyl und 2,2'-Bis(brommethyl)-1,1'-binapthyl.

2,2'-Bis(halogenmethyl)-1,1'-binaphthyle stellen als bifunktionelle Derivate wertvolle Ausgangsstoffe zur Herstellung einer größeren Zahl weiterer organischer Verbindungen dar.
Sie lassen sich beispielsweise durch übliche Reaktionen unter Austausch des Halogenatoms in die entsprechenden Alkohole, Nitrile oder Amine umwandeln. Diese Stoffe eignen sich ihrerseits als-bifunktionelle Verbindungen einerseits als Bausteine zur Herstellung von Kunststoffen wie Polyethern, Polyestern, Epoxidharzen oder Polyurethanen und andererseits als Komponenten für Schmiermittel, Additive, Stabilisatoren oder Antioxidantien.

2,2'-Bis(halogenmethyl)-1,1'-binaphthyle können auch unmittelbar zur Herstellung phosphororganischer Verbindungen verwendet werden.

So läßt sich 2,2'-Bis(brommethyl)-1,1'-binaphthyl als Ausgangsmaterial zur Herstellung von 2,2'-Bis(diphenylphosphinylmethyl)-1,1'-binaphthyl verwenden. Man setzt reines 2,2'-Bis(brommethyl)-1,1'-binaphthyl mit Diphenylphosphinigsäuremethylester um und erhält, wie aus JP 7 939 059 bzw. CA 91,91764 v hervorgeht, 2,2'-Bis(diphenylphosphinylmethyl)-1,1'-binaphthyl. Aus diesem Produkt läßt sich durch Reduktion 2,2'-Bis(diphenylphosphinomethyl)-1,1'-binaphthyl, ein zweizähniges Phosphin (Phosphan), herstellen. Dieses zweizähnige Phosphin findet als Ligand für Katalysatoren, beispielsweise bei der durch Metallkomplexe katalysierten Kupplung von Halogenaromaten, Verwendung. 2,2'-Bis(chlormethyl)-1,1'-binaphthyl wird hergestellt, indem man 2,2'-Bis(brommethyl)-1,1'-binaphthyl in Dimethylformamid mit Lithiumchlorid im Überschuß unter Chlor-Brom-Austausch umsetzt (Chong et al., J. Org, Chem. 58 (1993), 1266). Dieses Verfahren erfordert einen hohen Aufwand (Dimethylformamid als Lösungsmittel, Aufarbeitung in Wasser) und geht zudem von einem bereits dihalogenierten Material, nämlich dem entsprechenden dibromierten Produkt aus. Dieses Ausgangsmaterial ist nicht leicht zugänglich. Ferner führt die Umsetzung zu Lithiumchlorid und Lithiumbromid enthaltenden Abfallprodukten, die zu Schwierigkeiten bei der Aufarbeitung führen und Probleme bei der Entsorgung bereiten.

Zur Herstellung von 2,2'-Bis(brommethyl)-1,1'-binaphthyl geht man von 2,2'-Dimethyl-1,1'-binaphthyl aus, bromiert die Methylgruppen mit N-Bromsuccinimid und erhält 2,2'-Bis(brommethyl)-1,1'-binaphthyl. Bei dieser in der Literatur mehrfach beschriebenen, in Anwesenheit eines Radikalbildners in der Siedehitze, jedoch ohne Einwirkung von Licht durchgeführten Umsetzung findet in der Regel Tetrachlormethan als Lösungsmittel Anwendung (M.E. Jung et al., Tetrahedron Lett. 29 (1988) 6199; H.J. Bestmann et al., Chem. Ber. 107 (1.974) 2926; J.P. Mazaleyrat, Chem. Commun. 1985, 317; T. Hayashi et al., J. Am. Chem. Soc, l10 (1988) 8153).

Aus Tetrahedron Letters 30(27) 1989, Seiten 3513-3516 ist die Herstellung von 2,2'-Bis(brommethyl)-1,1'-binaphthyl durch Umsetzung von 2,2'-Dimethyl-1,1'-binaphthyl mit N-Bromsuccinimid in Tetrachlorkohlenstoff unter Einwirkung von Peroxid und Licht bekannt.

Wegen des vergleichsweise niedrigen Siedepunkts von Tetrachlormethan, der bei 76,5 "C liegt, läßt sich eine Umsetzung bei höheren Temperaturen nur unter Druck durchführen, was einen erhöhten technischen Aufwand erfordert. Darüber hinaus liegt die Ausbeute an 2,2'-Bis(brommethyl)-1,1'-binaphthyl mit ca. 60 % nicht sehr hoch.

Die nicht veröffentlichte europäische Patentanmeldung EP 93 116 788.6, die unter anderem auf die nicht veröffentlichte deutsche Patentanmeldung P 43 08 562.8 zurückgeht, beschreibt die Herstellung von 2,2'-Bis(brommethyl)-1,1'binaphthyl durch Umsetzung von 2,2'-Dimethyl-1,1'-binaphthyl mit N-Bromsuccinimid in Gegenwart von Benzoylperoxid, jedoch ohne Einwirkung von Licht, in siedendem Chlorbenzol (Siedepunkt: 132°C). Nach Beendigung der Umsetzung wird das Lösungsmittel verdampft, der Rückstand in Ethylacetat aufgenommen und zunächst mit 10 %iger Na₂S0₃-Lösung, dann mit gesättigter Na₂C0₃-Lösung und abschließend mit gesättigter NaCl-Lösung gewaschen. Nach Trocknen und Umkristallisieren erhält man 65 % Ausbeute. Dieses Verfahren erweist sich jedoch als aufwendig (Abdampfen des Lösungsmittel, Überführung des Rückstandes in ein anderes Lösungsmittel und dreifache Wäsche jeweils mit einer wäßrigen Natrium-Salz-Lösung), zudem läßt auch die Ausbeute noch zu wünschen übrig,

Es besteht daher an Bedarf, ein Verfahren zur Herstellung von 2,2'-Bis(halogen-methyl)-1,1'-binaphthylen zu entwickeln das sich nicht nur einerseits auf die Herstellung von 2,2'-Bis(chlormethyl)-1,1'-binaphthyl oder andererseits auf die Herstellung 2,2'-Bis(brommethyl)-1,1'-binaphthyl beschränkt, sondern sich auch allgemein anwenden läßt. Es soll die vorstehend geschilderten Nachteile nicht aufweisen, sich bei vergleichsweise niedrigen Temperaturen einfach und sicher ausführen lassen und das gewünschte Wertprodukt in guter Ausbeute und mit hoher Selektivität zugänglich machen.

Die Aufgabe wird gelöst durch ein Verfahren zur Herstellung von 2,2'-Bis(halogenmethyl)-1,1'-binaphthyl. Es ist dadurch gekennzeichnet, daß man 2,2'-Dimethyi-1,1'-binaphthyl in Anwesenheit von einem einfach oder mehrfach chlorierten Benzol oder einem Ester einer allphatischen Carbonsäure mit 1 bis 6 Kohlenstoffatomen und eines aliphatischen Alkohols mit 1 bis 4 Kohlenstoffatomen als Lösungsmittel mit einem Halogenierungsmittel unter Einwirkung von Licht einer Wellenlänge von 10⁻⁵ bis 10⁻⁸ m in Anwesenheit oder Abwesenheit eines Radikalbildners bei -10 bis 120°C umsetzt.

Aus dem Reaktionsablauf von Halogenierungen organischer Verbindungen ist bekannt, daß man eine Halogenierung in der Seitenkette eines Aromaten üblicherweise bei recht hohen Temperaturen (Siedehitze) in Gegenwart von Licht (Sonnenlicht) durchführt, während die Halogenierung des Kernes bei tiefen Temperaturen unter Verwendung eines Katalysators erfolgt.

Vor diesem Hintergrund ist es als überraschend anzusehen, daß die erfindungsgemäße Halogenierung unter Einwirkung von Licht bereits bei vergleichsweise niedrigen bis sehr niedrigen Temperaturen nicht in nennenswertem Umfange zu einer Halogenierung des aromatischen Kernes, sondern mit hoher Selektivität zu einer Halogenierung der Methylgruppen, das heißt der Seitenkette, im 2,2'-Dimethyl-1,1'-binaphthyl führt. Hierbei ist allerdings zu berücksichtigen, daß die jeweils gewählte Temperatur auch von der Reaktivität des jeweiligen Halogenierungsmittels abhängt. Ein relativ träges Halogenierungsmittel, beispielsweise N-Chlorsuccinimid, wird eine höhere Reaktionstemperatur als ein vergleichweises reaktives Halogenierungsmittel, wie Chlor, Brom oder N-Bromsuccinimid erforderlich machen.

Im allgemeinen spielt die verwendete Menge Lösungsmittel keine große Rolle. Sie sollte jedoch ausreichend bemessen sein. Im allgemeinen genügt es, 2,2'-Dimethyl-1, 1'-binaphthyl und das Lösungsmittel im Gewichtverhältnis 1: (3 bis 40), insbesondere 1: (4 bis 20), bevorzugt 1: (5 bis 15) zu verwenden.

Das erfindungsgemäße Verfahren wird in Anwesenheit von einem einfach oder mehrfach chlorierten Benzol oder einem Ester einer allphatischen Carbonsäure mit 1 bis 6 Kohlenstoffatomen und eines aliphatischen Alkohols mit 1 bis 4 Kohlenstoffatomen als Lösungsmittel durchgeführt. Diese Lösungsmittel sind unter den Reaktionsbedingungen, das heißt gegenüber der Einwirkung des Halogenierungsmittels unter den Reaktionsbedingungen, inert oder weitestgehend inert, wobei auch Mischungen derselben als Lösungsmittel eingetzt werden können.

Als Lösungsmittel eignet sich beispielsweise Chlorbenzol, ortho-, meta und para-Dichlorbenzol, Ameisensäuremethylester, Ameisensäureethylester, Essigsäuremethylester, Essigsäureethylester, Essigsäurepropylester, Essigsäurebutylester, Propionsäuremethylester, Propionsäureethylester, Propionsäurepropylester, Propionsäurebutylester, Buttersäuremethylester, Buttersäureethylester, Buttersäurepropylester und Buttersäurebutylester. Mischungen dieser Solventien lassen sich auch verwenden. Gut geeignet als Lösungsmittel sind Chlorbenzol und/oder Dichlorbenzol. Besonders geeignet ist Chlorbenzol. In einigen Fällen lassen sich auch Ester der vorstehend genannten Art, insbesondere Methylester und Ethylester aliphatischer Carbonsäuren mit 1 bis 3 Kohlenstoffatomen mit gutem Erfolg einsetzen.

Üblicherweise setzt man 2,2'-Dimethyl-1,1'-binaphthyl und das Halogenierungsmittel im Molverhältnis 1:(1,5 bis 2,5) ein. In vielen Fällen genügt es 2,2'-Dimethyl-1,1'-binaphthyl und das Halogenierungsmittel im Molverhältnis 1:(1,8 bis 2,3), bevorzugt 1:(1,9 bis 2,2) einzusetzen.

Man kann als Halogenierungsmittel ein Chlorierungsmittel oder ein Bromierungsmittel einsetzen. Es lassen sich die üblichen Chlorierungsmittel oder Bromierungsmittel verwenden. Beispiele für derartige Halogenierungsmittel sind Chlor, N-Chlorsuccinimid, Brom, N-Bromsuccinimid, 1,3-Dibrom-5,5-dimethylhydantoin oder Brommeldrumsäure.

In einer Reihe von Fällen hat es sich als günstig erwiesen, als Halogenierungsmittel N-Chlorsuccinimid oder N-Bromsuccinimid einzusetzen.

Verwendet man N-Chlorsuccinimid oder N-Bromsuccinimid als Halogenierungsmittel, so entsteht im Verlauf der Halogenierung sowohl aus dem N-Chlorsuccinimid als auch aus dem N-Bromsuccinimid jeweils Succinimid als Reaktionsprodukt. Das Succinimid kann, gegebenenfalls nach Abkühlen der das Reaktionsgemisch enthaltenden Lösung, durch Filtration abgetrennt werden. Eine andere Möglichkeit besteht darin, das gebildete Succinimid durch Extraktion mit Wasser abzutrennen. Üblicherweise setzt man hierfür 10 bis 100 Gew.-% Wasser, bezogen auf das Reaktionsgemisch ein.

Eine besonders einfache und zugleich wirksame Methode, Succinimid zu entfernen, besteht darin, das gebildete Succinimid in einem ersten Schritt durch Filtration und in einem zweiten Schritt durch Extraktion mit Wasser aus dem Reaktionsgemisch abzutrennen. Hierbei setzt man vergleichsweise wenig Wasser ein und erhält auch dementsprechend wenig Abwasser.

Das erfindungsgemäße Verfahren wird unter Einwirkung von Licht durchgeführt. Als Lichtquelle kann ein üblicher UV-Strahler, beispielsweise eine Tageslichtlampe, eine dotierte oder undotierte Quecksilberdampflampe oder Niederdruckquecksilberdampflampe, dienen.

Diese Lichtquellen weisen ein Spektrum von 10⁻⁵ bis 10⁻⁸, insbesondere 10⁻⁶ bis 2x10⁻⁷ m, auf. Darin sind auch die für die Umsetzung erforderlichen Lichtanteile, insbesondere UV-Anteile, enthalten.

Falls gewünscht, kann man die Umsetzung von 2,2'-Dimethyl-1,1'binaphthyl mit dem Halogenierungsmittel auch in Anwesenheit eines Radikalbildners durchführen. Es hat sich nämlich gezeigt, daß in einigen Fällen der Zusatz eines Radikalbildner (Radikalstarters) sich zusätzlich zu der Einwirkung von Licht günstig auswirken kann. Geeignete Radikalbildner sind die für eine radikalisch verlaufende Halogenierung üblichen Radikalbildner, beispielsweise organische Peroxide, organische Percarbonsäuren, organische Hydroperoxide oder organische Azoverbindungen.
Beispiele für geeignete Radikalbildner sind Benzoylperoxid, Benzoylperhexadecanoat und Azo-bis-isobutyronitril.

Nichtumgesetzten Radikalbildner kann man, beispielsweise durch Wäsche mit einer wäßrigen Na₂SO₃-Lösung, entfernen.

Man verwendet die Radikalbildner in üblichen Mengen. In der Regel genügt es, sie in einer Menge von 0,1 bis 5, insbesondere 0,5 bis 2 Gew.-%, bezogen auf umzusetzendes 2,2'-Dimethyl-1,1'-binaphthyl, einzusetzen.

Wie eingangs bereits erwähnt, führt man die Halogenierung bei -10 bis 120 °C durch. In vielen Fällen hat es sich als ausreichend erwiesen, die Halogenierung bei -5 bis 100, insbesondere 0 bis 80 °C ablaufen zu lassen.

In diesem Zusammenhang sei darauf hingewiesen, daß die Reaktionstemperatur, bei der die Halogenierung stattfinden soll, auch in nicht unerheblichem Umfange von dem verwendeten Halogenierungsmittel abhängt. Ein vergleichsweise reaktionsträges Halogenierungsmittel wird erst bei höheren Temperaturen als ein vergleichsweise reaktives Halogenierungsmittel mit dem 2,2'-Dimethyl-1,1'-binaphthyl reagieren.

Verwendet man ein Chlorierungsmittel als Halogenierungsmittel, so empfiehlt es sich, die Umsetzung bei 25 bis 120, insbesondere 50 bis 100 °C durchzuführen. Bei Verwendung von N-Chlorsuccinimid, das vergleichsweise reaktionsträge ist, arbeitet man bei 5 bis 120, insbesondere 70 bis 100 °C.

Verwendet man ein Bromierungsmittel als Halogenierungsmittel, so läßt sich das erfindungsgemäße Verfahren bei vergleichsweise recht tiefen Temperaturen durchführen. Im allgemeinen genügt es, die Bromierung bei -10 bis 80 °C vorzunehmen. Für eine Vielzahl von Fällen erweisen sich Reaktionstemperaturen von -5 bis 50, insbesondere 0 bis 40 °C als ausreichend.

Verwendet man Brom als Halogenierungsmittel, so empfiehlt es sich, bei 10 bis 80, insbesondere 25 bis 75, bevorzugt 35 bis 70 °C zu arbeiten. Bei Einsatz von N-Bromsuccinimid läßt sich die Halogenierung bei -10 bis 50, inbesondere -5 bis 40, bevorzugt 0 bis 30 °C mit gutem Erfolg durchführen.

Für eine eventuelle Weiterverarbeitung des nach der Halogenierung anfallenden Reaktionsgemisches kann es wünschenswert sein, das ursprünglich in der Stufe der Halogenierung verwendete Lösungsmittel durch ein weiteres Lösungsmittel zu ersetzen. Dies ist insbesondere dann notwendig, falls das ursprüngliche Lösungsmittel sich unter den Bedingungen der Weiterverarbeitung,

beispielsweise unter Einwirkung basischer Stoffe, nicht inert verhält. Man setzt in diesem Falle dem Reaktionsgemisch nach Halogenierung ein weiteres Lösungsmittel, das höher als das ursprünglich eingesetzte Lösungsmittel siedet, zu und destilliert anschließend das ursprünglich eingesetzte Lösungsmittel, je nach Wunsch und Bedarf, ganz oder teilweise ab.

Als weiteres Lösungsmittel kommen aromatische Kohlenwasserstoffe, beispielsweise Toluol, o-Xylol, m-Xylol, p-Xylol, Gemische dieser Xylole, Ethylbenzol und/oder Mesitylen und hochsiedende aliphatische Kohlenwasserstoffe, beispielsweise Petrolether mit einem Siedepunkt > 100 °C, Decalin, Ligroin und/oder Isooctan in Betracht. Will man den Lösungsmittelaustausch besonders schonend durchführen, kann man das ursprünglich eingesetzte Lösungsmittel als Azeotrop und/oder unter reduziertem Druck abdestillieren.

Beabsichtigt man das 2,2'-Bis(halogenmethyl)-1,1'-binaphthyl als Reinprodukt zu isolieren, so entfernt man gegebenenfalls gebildetes Succinimid, wie zuvor beschrieben, durch Filtration und Extraktion mittels Wasser, trocknet das Reaktionsgemisch und filtriert vom Trocknungsmittel ab. Anschließend entfernt man das ursprünglich eingesetzte Lösungsmittel unter reduziertem Druck. Bei Reingewinnung von 2,2'-Bis(brommethyl)-1,1'-binaphthyl sollte wegen der thermischen Labilität des Reaktionsgemisches, das sich bei Temperaturen ab 50 °C zu zersetzen beginnt, die Abtrennung des ursprünglich eingesetzten Lösungsmittels bei einer Sumpftemperatur < 50 °C erfolgen.
Bei Reingewinnung von 2,2'-Bis(chlormethyl)-1,1'-binaphthyl kann das ursprünglich eingesetzte Lösungsmittel bei höheren Temperaturen, beispielsweise bis etwa 70 °C abgetrennt werden, ohne daß nennenswerte Probleme auftreten. Das verbleibende, zumeist in öliger Konsistenz anfallende Rohprodukt wird in einem Lösungsmittel, beispielsweise Toluol, o-Xylol, m-Xylol, p-Xylol oder einem Gemisch dieser Lösungsmittel, aufgenommen und durch Umkristallisation gereinigt.

Die folgenden Beispiele belegen die Erfindung, ohne sie zu beschränken.

### Experimenteller Teil

### Beispiel 1

### Herstellung von 2,2'-Bis(chlormethyl)-1,1'-binaphthyl

In einem 4 l-Glaskolben werden unter Feuchtigkeitsausschluß 282.4 g (1.0 mol) 2,2'-Dimethyl-1,1'-binaphthyl und 280.4 g (2.1 mol) N-Chlorsuccinimid in 1.7 l Chlorbenzol suspendiert und 16 Stunden bei 70 °C mit einer UV-Tauchlampe belichtet. Man filtriert das ausgefallene Succinimid ab, extrahiert zweimal mit je 200 ml Wasser, trocknet mit Natriumsulfat, filtriert und engt man im Vakuum ein. Man erhält 350.0 g gelbbraunes Öl mit einer Zusammensetzung (gaschromatographische Analyse in Mol%) 55 % 2,2'-Bis(chlormethyl)-1,1'-binaphthyl, 28 % 2-Chlormethyl-2'-methyl-1,1'-binaphthyl und 10 % 2-Dichlormethyl-2'-chlormethyl-1,1'-binaphthyl und 7 % nicht näher identifizierte Produkte.

Der Umsatz beträgt, bezogen auf eingesetztes 2,2'-Dimethyl-1,1'-binaphthyl, 100 %, die Ausbeute an 2,2'-Bis(chlormethyl)-1,1'-binaphthyl beträgt 55 %, bezogen auf eingesetztes 2,2'-Dimethyl-1,1'-binaphthyl.

### Vergleichsversuch 1a

### Herstellung von 2,2'-Bis(chlormethyl)-1,1'-binaphthyl ohne Einwirkung von Licht

In einem 4 l-Glaskolben werden unter Feuchtigkeitsausschluß 282.4 g (1.0 mol) 2,2'-Dimethyl-1,1'-binaphthyl, 280.4 g (2.1 mol) N-Chlorsuccinimid und 500 mg Benzoylperoxid in 1.7 l Chlorbenzol suspendiert und 16 Stunden unter Rückfluß (132 °C) gerührt. Man filtriert das ausgefallene Succinimid ab, extrahiert zweimal mit je 200 ml Wasser und einmal mit 100 ml Na₂SO₃-Lösung, trocknet mit Natriumsulfat, filtriert und engt im Vakuum ein. Man erhält 354.0 g gelbbraunes Öl mit einer Zusammensetzung (gaschromatographische Analyse in Mol%) 38 % 2,2'-Bis(chlormethyl)-1,1'-binaphthyl, 35 % 2-Chlormethyl-2'-methyl-1,1'binaphthyl und 8 % 2-Dichlormethyl-2'-chlormethyl-1,1'-binaphthyl.

Der Umsatz beträgt bezogen auf eingesetztes 2,2'-Dimethyl-1,1'-binaphthyl, 96 % die Ausbeute an 2,2-Bis(chlormethyl)-1,1'-binaphthyl beträgt 37 %, bezogen auf eingesetztes 2,2'-Dimethyl-1,1'-binaphthyl.

### Vergleichsversuch 1b

### Herstellung von 2,2'-Bis(chlormethyl)-1,1'-binaphthyl unter Einwirkung von Licht, jedoch bei 130 °C

In einem 4 l-Glaskolben werden unter Feuchtigkeitsauschluß 282.4 g (1.0 mol) 2,2'-Dimethyl-1,1'-binaphthyl und 280.4 g (2.1 mol) N-Chlorsuccinimid in 1.7 l Chlorbenzol suspendiert und 16 Stunden bei 130 °C mit einer UV-Tauchlampe belichtet. Man filtriert das ausgefallene Succinimid ab, extrahiert zweimal mit je 200 ml Wasser, trocknet mit Natriumsulfat, filtriert und engt im Vakuum ein. Man erhält 354.0 g gelbbraunes Öl mit einer Zusammensetzung (gaschromatographische Analyse in Mol%) 47 % 2,2'-Bis(chlormethyl)-1,1'-binaphthyl, 22 % 2-Chlormethyl-2'-methyl-1,1'-binaphthyl und 12 % 2-Dichlormethyl-2'-chlormethyl-1,1'-binaphthyl und 19 % nicht näher identifizierter Produkte .
Der Umsatz beträgt, bezogen auf eingesetzes 2,2'-Dimethyl-1,1'-binapthyl 100 %, die Ausbeute an 2,2'-Bis(chlormethyl)-1,1'-binaphthyl beträgt 47 %, bezogen auf eingesetztes 2,2'-Dimethyl-1,1'-binaphthyl.

### Vergleichsversuch 1c

### Herstellung von 2,2'-Bis(chlormethyl)-1,1'-binaphthyl ohne Einwirkung von Licht (analog Beispiel 1, jedoch mit Radikalbildner)

In einem 4 l-Glaskolben werden unter Feuchtigkeitsausschluß 282.4 g (1.0 mol) 2,2'-Dimethyl-1,1'-binaphthyl, 280.4 g (2.1 mol) N-Chlorsuccinimid und 500 mg Benzoylperoxid in 1.7 l Chlorbenzol suspendiert und 16 Stunden bei 70 °C gerührt. Danach wird eine Probe entnommen, die gemäß Vergleichsversuch 1a aufgearbeitet wird.
Laut gaschromatographischer Analyse (in Mol%) des anfallenden Reaktionsgemisch beträgt der Umsatz, bezogen auf eingesetztes 2,2'-Dimethyl-1,1'-binaphthyl 40 %, die Ausbeute an 2,2'-Bis(chlormethyl)-1,1'-binaphthyl beträgt 18 %, bezogen auf eingesetztes 2,2'-Dimethyl-1,1'-binaphthyl.

### Beispiel 2

### Herstellung von 2,2'-Bis(brommethyl)-1,1'-binaphthyl

In einem 4 l Glaskolben werden unter Feuchtigkeitsausschluß 282,4 g (1 mol) 2,2'-Dimythyl-1,1'-binaphthyl und 373,9 g (2,1 mol) N-Bromsuccinmid mit 1,7 l Chlorbenzol versetzt und 8 Stunden bei 5 bis 10 °C unter Rühren mit einer UV-Tauchlampe belichtet. Man filtriert das ausgefallene Succinimid ab, extrahiert mit je 200 ml Wasser, trocknet mit Natriumsulfat und filtriert. Anschließend engt man im Vakuum bei 20 bis 30 °C ein und erhält 440 g eines gelbbraunen Öls mit einem Gehalt von 80 ± 2 Mol% 2,2'-Bis(brommethyl)-1,1'-binaphthyl. Aus Toluol kristallisieren 308 g farblose Kristalle mit einem Schmelzpunkt von 146 bis 149 °C.
Der Umsatz beträgt, bezogen auf eingesetztes 2,2'-Dimethyl-1,1'-binaphthyl 100 %, die Ausbeute an isoliertem 2,2'-Bis(brommethyl)-1,1'-binaphthyl beträgt 70 %, bezogen auf eingesetztes 2,2'-Dimethyl-1,1'-binaphthyl.

### Vergleichsversuch 2

### Herstellung von 2,2'-Bis(brommethyl)-1,1'-binaphthyl ohne Einwirkung von Licht entsprechend DE 43 08 562.8

Zu 8,5 g (30 mmol) 2,2'-Dimethyl-1,1'-binaphthyl in 100 ml Chlorbenzol gibt man unter Rückfluß bei Siedetemperatur (132 °C) portionsweise ein Gemisch aus 10,7 g (60 mmol) N-Bromsuccinimid und 100 mg Benzoylperoxid. Nach beendeter Zugabe wird noch 1 Stunde bei Siedetemperatur gerührt und das Lösungsmittel verdampft. Der Rückstand wird in 50 ml Ethylacetat aufgenommen und je 1 x mit 10 %iger Na₂SO₃-Lösung, gesättigter Na₂SO₃ und gesättigter NaCl-Lösung gewaschen. Nach dem Trocknen mit MgSO₄ wird eingeengt. Man erhält 13,2 g gelbes Öl. Aus Toluol kristallisieren 8,6 g farblose Kristalle mit Schmp. 147-149 °C.
Der Umsatz beträgt, bezogen auf eingesetztes 2,2'-Dimethyl-1,1'-binaphthyl, 100 %, die Ausbeute an isoliertem 2,2'-Bis(brommethyl-1,1'-binaphthyl beträgt 65 %, bezogen auf eingesetztes 2,2'-Dimethyl-1,1'-binaphthyl.

### Beispiel 3

### Herstellung von 2,2'-Bis(brommethyl)-1,1'-binaphthyl

In einem 4 l-Glaskolben mit nachgeschaltetem alkalischen Abluftwäscher werden unter Feuchtigkeitsausschluß und Stickstoffüberlagerung 282.4 g (1.0 mol) 2,2'-Dimethyl-1,1'-binaphthyl -binaphthyl in 2.5 l Chlorbenzol gelöst. Bei 25 °C und unter Belichtung mit einer UV-Tauchlampe tropft man 383.5 g (2.4 mol) Brom so zu, daß sich die Reaktionslösung sofort wieder entfärbt (Dauer 2 bis 3 Stunden). Nach einer Gesamtbelichtungsdauer von 5 Stunden extrahiert man nacheinander zweimal mit je 500 ml halbgesättigter NaHCO₃-Lösung und einmal mit 500 ml Wasser, trocknet mit Natriumsulfat, filtriert und engt im Vakuum ein. Man erhält 440.0 g gelbbraunes Öl mit einer Zusammensetzung (gaschromatographische Analyse in Mol%) 65 % 2,2'-Bis(brommethyl)-1,1'-binaphthyl, 12 % 2-Brommethyl-2'-methyl-1,1'-binaphthyl und 10 % 2-Dibrommethyl-2'-brommethyl-1,1'-binaphthyl.
Der Umsatz beträgt, bezogen auf eingesetztes 2,2'-Dimethyl-1,1'-binaphthyl 100 %, die Ausbeute an 2,2'-Bis(brommethyl)-1,1'-binaphthyl beträgt 65 %, bezogen auf eingesetztes 2,2'-Dimethyl-1,1'-binaphthyl.

## Patentansprüche

1. Verfahren zur Herstellung von 2,2'-Bis(halogenmethyl)-1,1'-binaphthyl, dadurch gekennzeichnet, daß man 2,2'-Dimethyl-1,1'-binapthyl in Anwesenheit von einem einfach oder mehrfach chlorierten Benzol oder einem Ester einer aliphatischen Carbonsäure mit 1 bis 6 Kohlenstoffatomen und eines aliphatischen Alkohols mit 1 bis 4 Kohlenstoffatomen als Lösungsmittel mit einem Halogenierungsmittel unter Einwirkung von Licht einer Wellenlänge von 10⁻⁵ bis 10⁻⁸ m in Anwesenheit oder Abwesenheit eines Radikalbildners bei -10 bis 120°C umsetzt.

2. Verfahren nach Anspruch 1, dadurch gekennzeichnet, daß man 2,2'-Dimethyl-1,1'-binaphthyl und das Lösungsmittel im Gewichtsverhältnis 1:(3 bis 40), insbesondere 1:(4 bis 20), bevorzugt 1:(5 bis 15) einsetzt.

3. Verfahren nach Anspruch 1 oder 2, dadurch gekennzeichnet, daß man 2,2'-Dimethyl-1,1'-binaphthyl in einem gegenüber den Reaktionsbedingungen inerten Lösungsmittel umsetzt.

4. Verfahren nach einem oder mehreren der Ansprüche 1 bis 3, dadurch gekennzeichnet, daß man als Lösungsmittel Chlorbenzol und/oder Dichlorbenzol einsetzt.

5. Verfahren nach einem oder mehreren der Ansprüche 1 bis 3, dadurch gekennzeichnet, daß man als Lösungsmittel Essigsäureethylester einsetzt.

6. Verfahren nach einem oder mehreren der Ansprüche 1 bis 5, dadurch gekennzeichnet, daß man 2,2'-Dimethyl-1,1'-binaphthyl und das Halogenierungsmittel im Molverhältnis 1:(1,5 bis 2,5), insbesondere 1:(1,8 bis 2,3), bevorzugt 1:(1,9 bis 2,2) einsetzt.

7. Verfahren nach einem oder mehreren der Ansprüche 1 bis 6, dadurch gekennzeichnet, daß man als Halogenierungsmittel ein Chlorierungsmittel oder Bromierungsmittel einsetzt.

8. Verfahren nach einem oder mehreren der Ansprüche 1 bis 7, dadurch gekennzeichnet, daß man als Halogenierungsmittel Chlor, N-Chlorsuccinimid, Brom, N-Bromsuccinimid, 1,3-Dibrom-5,5-dimethylhydantoin oder Brommeldrumsäure einsetzt.

9. Verfahren nach einem oder mehreren der Ansprüche 1 bis 8, dadurch gekennzeichnet, daß man als Halogenierungsmittel N-Chlorsuccinimid, Brom oder N-Bromsuccinimid einsetzt.

10. Verfahren nach einem oder mehreren der Ansprüche 1 bis 9, dadurch gekennzeichnet, daß man als Lichtquelle einen üblichen UV-Strahler verwendet.

11. Verfahren nach einem oder mehreren der Ansprüche 1 bis 10, dadurch gekennzeichnet, daß man als Radikalbildner ein organisches Peroxid, eine organische Percarbonsäure, ein organisches Hydroperoxid oder eine organische Azoverbindung einsetzt.

12. Verfahren nach einem oder mehreren der Ansprüche 1 bis 11, dadurch gekennzeichnet, daß man als Radikalbildner Benzoylperoxid, Benzoylperhexadecanoat oder Azo-bis-isobutyronitril einsetzt.

13. Verfahren nach einem oder mehreren der Ansprüche 1 bis 12, dadurch gekennzeichnet, daß man 2,2'-Dimethyl-1,1'-binaphthyl mit dem Halogenierungsmittel bei -5 bis 100, insbesondere 0 bis 80°C umsetzt.

14. Verfahren nach einem oder mehreren der Ansprüche 1 bis 13, dadurch gekennzeichnet, daß man 2,2'-Dimethyl-1,1'-binaphthyl mit einem Chlorierungsmittel bei 25 bis 120, insbesondere 70 bis 100°C umsetzt.

15. Verfahren nach einem oder mehreren der Ansprüche 1 bis 14, dadurch gekennzeichnet, daß man 2,2'-Dimethyl-1,1-binaphthyl mit einem Bromierungsmittel bei -10 bis 80, insbesondere -5 bis 50, bevorzugt 0 bis 40°C umsetzt.

## Claims

1. A process for preparing 2,2'-bis(halomethyl)-1,1'-binaphthyl, which comprises reacting 2,2'-dimethyl-1,1'-binaphthyl in the presence of a monochlorinated or poly-chlorinated benzene or an ester of an aliphatic carboxylic acid having from 1 to 6 carbon atoms and an aliphatic alcohol having from 1 to 4 carbon atoms as solvent with a halogenating agent under the action of light having a wavelength of from 10⁻⁵ to 10⁻⁸ m in the presence or absence of a free-radical former at from -10 to 120°C.

2. The process as claimed in claim 1, wherein 2,2'-dimethyl-1,1'-binaphthyl and the solvent are used in a weight ratio of 1:(3 to 40), in particular 1:(4 to 20), preferably 1:(5 to 15).

3. The process as claimed in claim 1 or 2, wherein 2,2'-dimethyl-1,1'-binaphthyl is reacted in a solvent which is inert to the reaction conditions.

4. The process as claimed in one or more of claims 1 to 3, wherein the solvent used is chlorobenzene and/or dichlorobenzene.

5. The process as claimed in one or more of claims 1 to 3, wherein the solvent used is ethyl acetate.

6. The process as claimed in one or more of claims 1 to 5, wherein 2,2'-dimethyl-1,1'-binaphthyl and the halogenating agent are used in a molar ratio of 1:(1.5 to 2.5), in particular 1:(1.8 to 2.3), preferably 1:(1.9 to 2.2).

7. The process as claimed in one or more of claims 1 to 6, wherein the halogenating agent used is a chlorinating agent or brominating agent.

8. The process as claimed in one or more of claims 1 to 7, wherein the halogenating agent used is chlorine, N-chlorosuccinimide, bromine, N-bromosuccinimide, 1,3-dibromo-5,5-dimethylhydantoin or brominated Meldrum's acid.

9. The process as claimed in one or more of claims 1 to 8, wherein the halogenating agent used is N-chlorosuccinimide, bromine or N-bromosuccinimide.

10. The process as claimed in one or more of claims 1 to 9, wherein the light source used is a customary UV irradiator.

11. The process as claimed in one or more of claims 1 to 10, wherein the free-radical former used is an organic peroxide, an organic percarboxylic acid, an organic hydroperoxide or an organic azo compound.

12. The process as claimed in one or more of claims 1 to 11, wherein the free-radical former used is benzoyl peroxide, benzoyl perhexadecanoate or azobisisobutyronitrile.

13. The process as claimed in one or more of claims 1 to 12, wherein 2,2'-dimethyl-1,1'-binaphthyl is reacted with the halogenating agent at from -5 to 100°C, in particular from 0 to 80°C.

14. The process as claimed in one or more of claims 1 to 13, wherein 2,2'-dimethyl-1,1'-binaphthyl is reacted with a chlorinating agent at from 25 to 120°C, in particular from 70 to 100°C.

15. The process as claimed in one or more of claims 1 to 14, wherein 2,2'-dimethyl-1,1'-binaphthyl is reacted with a brominating agent at from -10 to 80°C, in particular from -5 to 50°C, preferably from 0 to 40°C.

## Revendications

1. Procédé de préparation de 2,2'-bis-(halogénométhyl)-1,1'-binaphtyle, caractérisé en ce que l'on fait réagir le 2,2'-diméthyl-1,1'-binaphtyle en présence d'un ester d'un acide aliphatique carboxylique avec 1 à 6 atomes de carbone ou d'un benzène chloré une ou plusieurs fois et d'un alcool aliphatique avec 1 à 4 atomes de carbone en tant que solvant, avec un agent d'halogénation sous l'effet de la lumière d'une longueur d'onde de 10⁻⁵ à 10⁻⁸ m en présence ou en l'absence d'un formateur de radicaux, à une température de -10 à 120 °C.

2. Procédé selon la revendication 1, caractérisé en ce que l'on utilise le 2,2'-diméthyl-1,1'-binaphtyle et le solvant dans un rapport molaire de 1:(3 à 40), en particulier de 1:(4 à 20), de préférence de 1:(5 à 15).

3. Procédé selon la revendication 1 ou 2, caractérisé en ce que l'on fait réagir le 2,2'-diméthyl-1,1'-binaphtyle dans un solvant inerte sous les conditions réactionnelles.

4. Procédé selon une ou plusieurs des revendications 1 à 3, caractérisé en ce que l'on utilise comme solvant le chlorobenzène et/ou de dichlorobenzène.

5. Procédé selon une ou plusieurs des revendications 1 à 3, caractérisé en ce que l'on utilise comme solvant l'acétate d'éthyle.

6. Procédé selon une ou plusieurs des revendications 1 à 5, caractérisé en ce que l'on utilise le 2,2'-diméthyl-1,1'-binaphtyle et l'agent d'halogénation dans un rapport molaire de 1:(1,5 à 2,5), en particulier de 1:(1,8 à 2,3), de préférence de 1:(1,9 à 2,2).

7. Procédé selon une ou plusieurs des revendications 1 à 6, caractérisé en ce que l'on utilise comme agent d'halogénation un agent de chloration ou un agent de bromation.

8. Procédé selon une ou plusieurs des revendications 1 à 7, caractérisé en ce que l'on utilise comme agent d'halogénation le chlore, le N-chlorosuccinimide, le brome, le N-bromosuccinimide, la 1,3-dibromo-5,5-diméthylhydantoïne ou l'acide bromomeldrumique.

9. Procédé selon une ou plusieurs des revendications 1 à 8, caractérisé en ce que l'on utilise comme agent d'halogénation le N-chlorosuccinimide, le brome ou le N-bromosuccinimide.

10. Procédé selon une ou plusieurs des revendications 1 à 9, caractérisé en ce que l'on utilise comme source de lumière des émetteurs d'UV usuels.

11. Procédé selon une ou plusieurs des revendications 1 à 10, caractérisé en ce que l'on utilise comme formateur de radicaux un peroxyde organique, un acide percarbonique organique, un hydroperoxyde organique ou un composé azoïque organique.

12. Procédé selon une ou plusieurs des revendications 1 à 11, caractérisé en ce que l'on utilise comme formateur de radicaux le peroxyde de benzoyle, le perhexadécanoate de benzoyle ou l'azo-bis-butyronitrile.

13. Procédé selon une ou plusieurs des revendications 1 à 12, caractérisé en ce que l'on fait réagir le 2,2'-diméthyl-1,1'-binaphtyle avec l'agent d'halogénation à une température de -5 à 100, en particulier de 0 à 80 °C.

14. Procédé selon une ou plusieurs des revendications 1 à 13, caractérisé en ce que l'on fait réagir le 2,2'-diméthyl-1,1'-binaphtyle avec un agent de chloration à une température de 25 à 120, en particulier de 70 à 100 °C.

15. Procédé selon une ou plusieurs des revendications 1 à 14, caractérisé en ce que l'on fait régir le 2,2'-diméthyl-1,1'-binahtyle avec un agent de bromation à une température de -10 à 80, en particulier de -5 à 50, de préférence de 0 à 40 °C.
